(19) Europäisches Patentamt
European Patent Office
Office européen des brevets

(11) EP 1 606 246 B1

(12) # EUROPÄISCHE PATENTSCHRIFT

(45) Veröffentlichungstag und Bekanntmachung des Hinweises auf die Patenterteilung:
**19.05.2010 Patentblatt 2010/20**

(21) Anmeldenummer: **04718192.0**

(22) Anmeldetag: **06.03.2004**

(51) Int Cl.:
***C07C 253/30*** (2006.01) ***C07C 255/23*** (2006.01)

(86) Internationale Anmeldenummer:
**PCT/EP2004/002311**

(87) Internationale Veröffentlichungsnummer:
**WO 2004/083165 (30.09.2004 Gazette 2004/40)**

(54) **VERFAHREN ZUR HERTELLUNG VON 2-CYAN-3-HYDROXY-N-(PHENYL)BUT-2-ENAMIDEN**

METHOD FOR PRODUCING 2-CYAN-3-HYDROXY-N-(PHENYL)BUT-2-ENE AMIDES

PROCEDE DE PRODUCTION DE 2-CYAN-3-HYDROXY-N-(PHENYL)BUT-2-ENE-AMIDES

(84) Benannte Vertragsstaaten:
**AT BE BG CH CY CZ DE DK EE ES FI FR GB GR HU IE IT LI LU MC NL PL PT RO SE SI SK TR**
Benannte Erstreckungsstaaten:
**LT LV MK**

(30) Priorität: **18.03.2003 DE 10311763**

(43) Veröffentlichungstag der Anmeldung:
**21.12.2005 Patentblatt 2005/51**

(73) Patentinhaber: **Sanofi-Aventis Deutschland GmbH**
**65929 Frankfurt am Main (DE)**

(72) Erfinder:
- **HACHTEL, Jochen**
**65929 Frankfurt (DE)**
- **NEISES, Bernd**
**77652 Offenburg (DE)**
- **SCHWAB, Wilfried**
**82061 Neuried (DE)**
- **UTZ, Roland**
**65926 Frankfurt am Main (DE)**
- **ZAHN, Martin**
**65926 Frankfurt am Main (DE)**

(56) Entgegenhaltungen:
**EP-A- 0 257 882 US-A- 5 519 042**
**US-A- 5 700 822**

- **SUTAPA GHOSH ET AL: "Three leflunomide analogs" ACTA CRYSTALLOGR. SECT. C CRYST. STRUCT. COMMUN., Bd. 56, Nr. 10, 2000, Seiten 1254-1257, XP002959255**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft ein Verfahren zur Herstellung von 2-Cyan-3-hydroxy-N-(phenyl)but-2-enamiden aus 2-Cyan-N-(phenyl)acetamid, insbesondere die Herstellung von 2-Cyan-3-hydroxy-N-[(4-trifluormethyl)-phenyl]but-2-enamid aus 2-Cyan-N-(4-trifluormethyl-phenyl)acetamid.

Die Verbindung 2-Cyan-3-hydroxy-N-[(4-trifluormethyl)phenyl]but-2-enamid ist bekannt (US 5,679,709). Verfahren zur Herstellung der 2-Cyan-3-hydroxy-N-[(4-trifluormethyl)phenyl]but-2-enamid werden beispielsweise in US 5,519,042 oder US 5,700,822 beschrieben. Nachteile der bekannten Verfahren sind eine geringe Ausbeute und eine geringe Reinheit.

Es wurde nun gefunden, dass sich 2-Cyan-3-hydroxy-N-[phenyl]but-2-enamide aus 2-Cyan-N-(phenyl)acetamid in Anwesenheit von Essigsäureanhydrid und Natriumhydroxid herstellen lassen.

Die Erfindung betrifft daher ein Verfahren zur Gewinnung der Verbindung der Formel I

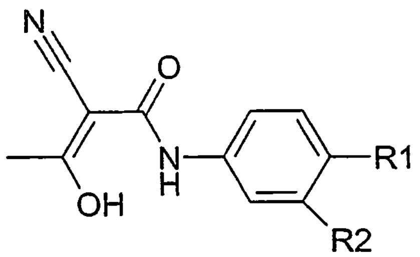

(I)

wobei

R1 für

a) $-CF_3$,
b) $-O-CF_3$,
c) $-S-CF_3$,
d) -OH,
e) $-NO_2$,
f) Halogen,
g) Benzyl,
h) Phenyl,
i) -O-Phenyl,
k) -CN,
l) -O-Phenyl, ein oder mehrfach substituiert mit
1) $(C_1-C_4)$-Alkyl,
2) Halogen,
3) $O-CF_3$ oder
4) $-O-CH_3$, steht, und

R2 für

a) $(C_1-C_4)$-Alkyl,
b) Halogen oder
c) Wasserstoffatom steht,

das dadurch gekennzeichnet ist, dass man eine Verbindung der Formel II,

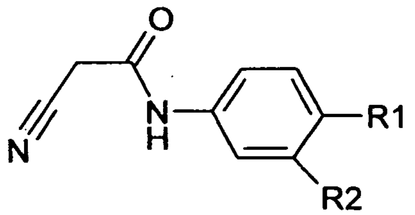

(II)

worin R1 und R2 wie oben definiert sind,
in Anwesenheit von Essigsäureanhydrid, mindestens einem Lösungsmittel und mindestens einer Base aus der Gruppe
Alkalihydroxide wie Natriumhydroxid oder Kaliumhydroxid, Ätznatron in fester Form oder in Form von Laugen,
Amide wie Natriumamid,
Alkoholate wie Natriummethylat oder Kalium-tert.-butylat,
metallorganische Verbindungen wie n-Butyllithium oder
Mischungen der Basen, umsetzt und anschließend die gebildete Verbindung der Formel I isoliert.

Die Erfindung betrifft ferner ein Verfahren zur Gewinnung der Verbindung der Formel III

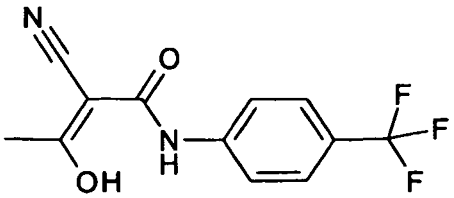

(III)

das dadurch gekennzeichnet ist, dass man eine Verbindung der Formel IV

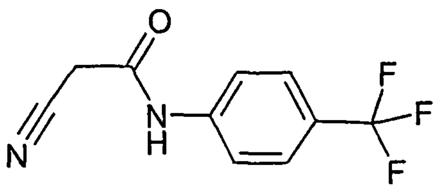

(IV)

in Anwesenheit von Wasser, Natriumhydroxid, Essigsäureanhydrid und einem zusätzlichen Lösungsmittel umsetzt und anschließend die gebildete Verbindung der Formel III isoliert.

Bei der Herstellung der Verbindung der Formel I geht man so vor, dass zunächst die Verbindung der Formel II oder (2-Cyan-N-(4-trifluormethyl-phenyl)acetamid) in einem Lösungsmittel vorgelegt wird und die erhaltene Lösung oder Suspension abgekühlt wird. Danach erfolgt die Zugabe von wässrigen Natriumhydroxid und Essigsäureanhydrid und das erhaltene Reaktionsgemisch wird unter Kühlung gerührt oder geschüttelt.

Nach einer entsprechenden Reaktionszeit wird die Verbindung der Formel I mit einer Säure ausgefällt. Die Isolierung der Verbindung der Formel I erfolgt beispielsweise durch Kristallisation oder Extraktion beispielsweise mit Ethylacetat oder Toluol. Die Kristallisation wird durch Kühlen der Suspension oder weiteren Verdampfen der Lösemittel gefördert.

Unter dem Begriff "Lösungsmittel" werden beispielsweise Wasser, organische Lösungsmittel beispielsweise:

ketonische Lösungsmittel wie Aceton, Methylethylketon oder Methylisobutylketon;
halogenierte Kohlenwasserstoffe wie Dichlormethan;
Alkohole wie Ethanol, Isopropanol oder n-Butanol;
Ether wie Diisopropylether, Diethoxymethan oder Diethylenglykoldimethylether;
Kohlenwasserstoffen wie Toluol;
Ester wie Ethylacetat;
aprotische Lösungsmittel wie Dimethylformamid, Dimethylacetamid, Dimethylsulfoxid oder N-Methylpyrrolidinon oder
Mischungen der genannten Lösungsmittel verstanden, oder Phasentransferkatalysatoren wie Quaternäre Ammonium- oder Phosphoniumsalze, beispielsweise Dimethylditetradecylammoniumbromid, Benzyl-triethyl-ammoniumchlorid, Alliquat® 336 (3-Methyl-trioctylammoniumchlorid), Tetrabutylammoniumhydrogensulfat, oder Tetrabutylphosphoniumchlorid;
Cronenether oder Cryptanden wie 18-Krone-6 oder Cryptand 222 [= 4,7,13,16,21,24-HEXAOXA-1,10-DIAZABICYCLO-(8.8.8)HEXACOSANE]; oder
Polyethylenglykole zum Einsatz kommen können, was besonders bei wenig wassermischbaren Lösemitteln wie Toluol von Vorteil ist.

Unter dem Begriff "Basen" versteht man Alkalihydroxide beispielsweise Natriumhydroxid oder Kaliumhydroxid, Ätznatron in fester Form oder in Form von Laugen unterschiedlicher Konzentration, Amide z.B. Natriumamid; Alkoholate z.B. Natriummethylat, Kalium-tert.-butylat, metallorganische Verbindungen z.B. n-Butyllithium oder Mischungen der genannten anorganische Basen verstanden. Das in der Lauge vorhandene Wasser geht dann bei der Bereitung der Reaktionsmischung in die Berechnung mit ein.

Unter dem Begriff "Halogen" werden Fluor, Chlor, Brom und Jod verstanden. Unter dem Begriff "-($C_{1-}C_4$)-Alkyl" werden Kohlenwasserstoffreste wie Methyl, Ethyl, Propyl, n-Butyl oder Iso-Butyl verstanden.

Geeignete Säuren sind beispielsweise Salzsäure, Schwefelsäure, Salpetersäure oder Phosphorsäure oder Mischungen der Säuren.

Vorzugsweise verwendet man für die erfindungsgemäße Reaktion auf 100 Mol der Verbindung der Formel II oder IV 150 Mol bis 300 Mol Essigsäureanhydrid und 100 Mol bis 550 Mol Natriumhydroxid.

Die verwendete Menge am Lösungsmittel beträgt im allgemeinen von 3 kg bis 11 kg pro kg der Verbindung der Formeln II oder IV, bevorzugt von 4 kg bis 6 kg.

Die Reaktionszeit liegt im allgemeinen zwischen wenigen Minuten und 24 Stunden, bevorzugt bei 1 bis 3 Stunden je nach Zusammensetzung des Gemisches und des gewählten Temperaturbereiches.

Die Reaktionstemperatur beträgt von -5 °C bis 50 °C, bevorzugt von 0 °C bis 30°C, insbesondere 10°C.

Der Restgehalt am Ausgangssubstrat der Verbindung der Formel II oder 2-Cyan-N-(4-trifluormethyl-phenyl) acetamid konnte im isolierten 2-Cyan-3-hydroxy-N-[(4-trifluormethyl)-phenyl]but-2-enamid unter ein Gehalt von 0,5 % verringert werden.

Die Ausgangssubstanz für die erfindungsgemäße Reaktion ist nach literaturbekannten Verfahren herstellbar, beispielsweise DE 1 900 947.

Das Verfahrensprodukt ist biologisch aktiv und eignet sich beispielsweise zur Behandlung von rheumatoider Arthritis oder Multipler Sklerose.

Vorteilhaft sind im erfindungsgemäßen Verfahren die sehr kurzen Reaktionszeiten, der Wegfall von zusätzlichen Reinigungsschritten, die hohen Ausbeuten und die hohe Reinheit des hergestellten Produkts. Vorteil des erfindungsgemäßen Verfahrens ist die im wesentlichen vollständige Umsetzung zur Verbindung der Formel I oder 2-Cyan-3-hydroxy-N-[(4-trifluormethyl)-phenyl]but-2-enamid und ein Gesamtnebenproduktgehalt von weniger als 1 %.

Beispiel 1

Herstellung von 2-Cyan-3-hydroxy-N-[(4-trifluormethyl)-phenyl]but-2-enamid

In einen Teflonreaktor mit Fritte wurden 2,5 g 2-Cyan-N-(4-trifluormethyl-phenyl)acetamid und 0,127 g Dimethylditetradecylammoniumbromid eingewogen. Der Reaktor wurde mit Argon überlagert und mit 15 ml Diethoxymethan befüllt. Die Schüttelung wurde angestellt und es wurde auf 15 °C gekühlt. Es wurden 1,73 ml wässrige, 50 %ige NaOH manuell zugegeben und innerhalb von 5 bis 20 min wurden insgesamt 2,12 ml Essigsäureanhydrid zudosiert. Danach erfolgte mehrfache eine HPLC-Kontrolle der Reaktionsmischung. Die Suspension wurde nach der Essigsäureanhydrid-Zugabe noch 8,5 Stunden (h) geschüttelt.

Danach wurde der Reaktor auf 5 °C abgekühlt. Innerhalb von 10 (Minuten) min wurden je 3,20 ml Wasser und 2,90 ml HCl, 37 % zugegeben, wobei die Reaktorinnentemperatur auf 5°C gehalten wurde. Bei dieser Temperatur wurde 1 h geschüttelt. Im Anschluß wurden in jeden Reaktor 13,0 ml Wasser dosiert, auf 10 °C erwärmt und 1 h geschüttelt. Die weiße Suspension wurden mit Argon abgedrückt. Die zurückgebliebenen Feststoffe wurden 3 mal mit je 15,0 ml Wasser gewaschen, bei 45 °C und 150 mbar bis zur Gewichtskonstanz getrocknet, ausgewogen und per HPLC analysiert..

HPLC Methode für 2-Cyan-3-hydroxy-N-[(4-trifluormethyl)-phenyl]but-2-enamid

| | | |
|---|---|---|
| Prüftechnik: | Flüssigchromatographie (Ph.Eur.) | |
| Gerät: | Flüssigchramatograph:<br>Waters Alliance Separations Module 2690 | |
| Säule: | Material:<br>Länge:<br>Innerer Durchmesser: | Edelstahl<br>100 mm<br>4,6mm |
| Stationäre Phase: | Waters Symmetry-C18<br>Korngröße 3,5 µm | |
| Mobile Phase: | Acetonitril:<br>Wasser:<br>Triethylamin:<br>mit $H_3PO_4$ 85 % auf pH 6,0 einstellen | 350 mL<br>650 mL<br>5 mL |
| Injektionsvolumen: | 10 µL | |
| Probengeber Temperatur: | 12°C | |
| Säulentemperatur: | 20°C | |
| Fluß:<br>Laufzeit: | 0,8 ml/min<br>45 min | |
| Detektion:<br>Standardlösung: | UV/Vis, 210 nm<br>14,0 mg Referenzstandard werden in 2 mL Acetonitril beschallt und auf 20 mL aufgefüllt. | |
| Prüflösung: | 12,5 mg Substanz werden mit Acetonitril R zu 25 mL gelöst. | |

| Retentionszeiten (min): | absolut | relativ |
|---|---|---|
| 2-Cyan-3-hydroxy-N-[(4-trifluormethyl)-phenyl]but-2-enamid: | 6,4 ± 10% | 1,0 |
| 2-Cyan-N-(4-trifluormethyl-phenyl)acetamid: | 12,8 ± 10% | 2,0 ± 10% |
| 4-Trifluormethylnilin: | 14,1 ± 10% | 2,2 ± 10% |

Berechnung:

$$\frac{FN \cdot 100}{\sum F} = \text{\% Nebenprodukt}$$

FN = Peakfläche des jeweiligen Nebenproduktes im Chromatogramm der Prüflösung

ΣF = Summe aller Peakflächen im Chromatogramm der Prüflösung außer des Einspritzpeaks

Beispiele 2 bis 7

Analog zu Beispiel 1 wurden die Versuche in Tabelle 1 durchgeführt.

Tabelle. 1: Verschiedene Lösungsmittelgemische

| Beispiel | PTC(1) | Lösemittel | Volumen [ml] | Moläquivalente PTC | Ausbeute [% der Theorie] |
|---|---|---|---|---|---|
| 2 | Dimethyl-ditetradecyl-ammonium-bromid | Toluol | 30 | 0,1 | 48,3 |
| 3 | Dimethyl-ditetradecyl-ammonium-bromid | Toluol | 30 | 0,05 | 53,6 |
| 4 | Dimethyl-ditetradecyl-ammonium-bromid | Diethoxy-methan | 25 | 0,1 | 65,4 |
| 5 | Benzyl-triethyl-ammonium-chlorid | Diethoxy-methan | 30 | 0,05 | 73,7 |
| 6 | Dimethyl-ditetradecyl-ammonium-bromid | Diethoxy-methan | 30 | 0,05 | 79,4 |
| 7 | Benzyl-triethyl-ammonium-chlorid | Diethoxy-methan | 15 | 0,1 | 77,8 |
| (1) PTC bedeutet Phasen-Transfer-Katalysator | | | | | |

Beispiel 8:

In einen Teflonreaktor mit Fritte wurden 2,5 g 2-Cyan-N-(4-trifluormethyl-phenyl)acetamid und 15 ml Aceton gefüllt. Die Schüttelung wurde angestellt und auf 15 °C gekühlt. Es wurden 1,73 ml wässrige, 50 %ige NaOH manuell zugegeben und innerhalb von 5 bis 20 min wurden 2,12 ml Essigsäureanhydrid zudosiert, dabei erfolgte mehrfache Probennahme und HPLC-Kontrolle. Die Suspension wurde nach der Essigsäureanhydrid-Zugabe noch 8,5 h geschüttelt und auf 5°C gekühlt. Innerhalb von 10 min wurden je 3,20 ml Wasser und 2,90 ml HCl, 37 % zugegeben, wobei die Reaktorinnentemperatur auf 5 °C gehalten wurde. Bei dieser Temperatur wurde 1 h geschüttelt. Im Anschluß wurden 13,0 ml Wasser zudosiert, auf 10°C erwärmt und 1 h geschüttelt. Die weißen Suspensionen wurden mit Argon abgedrückt. Die zurückgebliebenen Feststoffe wurden 3 mal mit je 15,0 ml E-Wasser gewaschen, bei 45°C und 150 mbar bis zur Gewichtskonstanz getrocknet, ausgewogen und per HPLC analysiert. Ausbeute 2,76 g (92% der Theorie, HPLC Reinheit 98,3)

Beispiel 9:

In einen 200 ml Vierhalskolben wurden 9,1 g 2-Cyan-N-(4-trifluormethyl-phenyl)acetamid eingewogen und dann durch Zugabe von 49 ml Methylisobutylketon suspendiert (leicht gelbe dünne Suspension), das anschließend auf 10°C abgekühlt wurde. Bei dieser Temperatur wurden 17,8 ml 33%ige NaOH direkt aus dem Meßzylinder zugegeben. Dabei stieg die Temperatur auf 12°C an und es entstand eine schwer rührbare kremefarbige Suspension. Diese wurde 10 min kräftig gerührt. Danach werden innerhalb von 1 h und 20 min 9,7 ml Essigsäureanhydrid bei 7 bis 12°C zugetropft. Hierbei ging die zähflüssige Suspension in eine schwach trübe orange Lösung über, aus der nach 50 min Zutropfen (etwa 7 ml zugetropft) ein Feststoff auskristallisierte, wodurch ein spontaner Temperaturanstieg zu beobachten war (Maximum mehr als 12°C). Nach Probennahme und HPLC-Analyse zeigten sich dabei noch etwa 1,29 Fl.-% der Ausgangssubstanz 2-Cyan-N-(4-trifluormethyl-phenyl)acetamid in der Reaktionsmischung. Das Zutropfen wurde nicht unterbrochen. Am Ende des Zutropfens nach 1 h und 20 min wurde nach HPLC-Analyse der vollständiger Umsatz erreicht (etwa 0,13 Fl.-% von 2-Cyan-N-(4-trifluor-methyl-phenyl)acetamid).

Es wurde 50 min weiter gerührt und dabei auf 3 bis 5°C abgekühlt. Dann wurden bei dieser Temperatur 11,5 ml Wasser innerhalb von 10 min zugetropft, das Gemisch hatte einen pH-Wert von 7,1. Im Anschluss wurden 16 ml 37%ige HCl innerhalb von 1 h zugetropft, so dass bei gleichbleibender Temperatur ein pH-Wert von 1,1 erreicht wurde.

Nach weiteren Rühren für 25 min war der pH-Wert noch immer bei 1,1 (cremefarbige, nicht homogene rührbare Suspension, bereits nach etwa 7 ml HCl). Innerhalb der nächsten 20 min wurden 47,5 ml Wasser zugetropft, wobei die Temperatur auf 10 °C anstieg. Der pH stieg auf 1,7. Anschließend wurde 40 min nachgerührt. Danach wurde die Suspension abgesaugt und der Rückstand mit 5 mal 30 ml Wasser Chlorid-frei gewaschen. Man erhielt einen cremefarbenen Feststoff, der bei 40 °C unter verminderten Druck getrocknet wurde. Ausbeute: 9,8 g (91% der Theorie, HPLC Reinheit 99,0)

Analog zu den Beispielen 8 und 9 wurden die Reaktionen gemäß der Tabelle 2 durchgeführt.

Tabelle 2

| Beispiel | Lösungsmittel | Äquivalente Essigsäure-anhydrid | Äquivalente NaOH | Ausbeute [% der Theorie] | Produkt (HPLC-Reinheit) |
|---|---|---|---|---|---|
| 10 | n-Butanol | 1,5 | 2,25 | 47 | 66,7 |
| 11 | i-Propanol | 1,5 | 2,25 | 63 | 69,3 |
| 12 | Aceton | 1,5 | 2,25 | 92 | 98,3 |
| 13 | Methyl-isobutylketon | 1,5 | 2,25 | 74 | 93,8 |
| 14 | Methyl-isobutylketon | 1,5 | 2,26* | 54 | 84,1 |
| 15 | Methyl-isobutylketon | 2,5 | 5,00** | 91 | 99,0 |
| 16 | N-Methylpyrrolidon | 1,5 | 2,26 | 58 | 66,5 |
| * 30%ige NaOH ** 33%ige NaOH | | | | | |

## Patentansprüche

**1.** Verfahren zur Gewinnung der Verbindung der Formel I

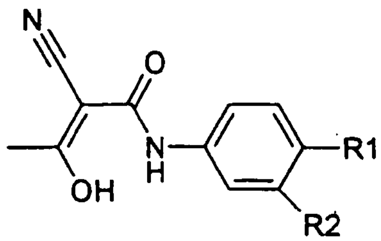

(I)

wobei

R1 für

a) $-CF_3$,
b) $-O-CF_3$,
c) $-S-CF_3$,
d) -OH,
e) $-NO_2$,
f) Halogen,
g) Benzyl,
h) Phenyl,
i) -O-Phenyl,

k) -CN,
l) -O-Phenyl, ein oder mehrfach substituiert mit
1) ($C_1$-$C_4$)-Alkyl,
2) Halogen,
3) O-$CF_3$ oder
4) -O-$CH_3$, steht, und

R2 für

a) ($C_1$-$C_4$)-Alkyl,
b) Halogen oder
c) Wasserstoffatom steht,

das **dadurch gekennzeichnet ist, dass** man eine Verbindung der Formel II,

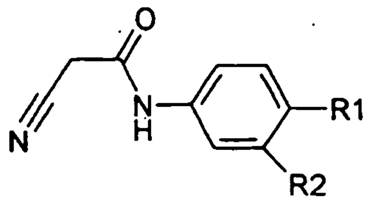

(II)

worin R1 und R2 wie oben definiert sind,
in Anwesenheit von Essigsäureanhydrid, mindestens einem Lösungsmittel und mindestens einer Base aus der Gruppe
Alkalihydroxide wie Natriumhydroxid oder Kaliumhydroxid, Ätznatron in fester Form oder in Form von Laugen,
Amide wie Natriumamid,
Alkoholate wie Natriummethylat oder Kalium-tert.-butylat,
metallorganische Verbindungen wie n-Butyllithium oder
Mischungen der Basen, umsetzt und anschließend die gebildete Verbindung der Formel I isoliert.

**2.** Verfahren gemäß Anspruch 1, **dadurch gekennzeichnet, dass** man eine Verbindung der Formel III

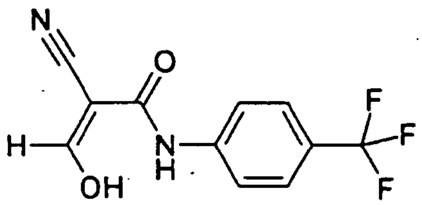

(III)

aus einer Verbindung der Formel IV

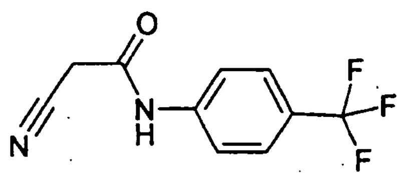

(IV)

herstellt und anschließend die gebildete Verbindung der Formel III isoliert.

**3.** Verfahren gemäß Anspruch 1 oder 2, **dadurch gekennzeichnet, dass** man als Lösungsmittel Wasser, ketonische Lösungsmittel wie Aceton, Methylethylketon, Diethoxymethan oder Methylisobutylketon;

halogenierte Kohlenwasserstoffe wie Dichlormethan;
Alkohole wie Ethanol, isopropanol oder n-Butanol;
Ether wie Diisopropylether, Diethoxymethan oder Diethylenglykoldimethylether;
Kohlenwasserstoffen wie Toluol;
Ester wie Ethylacetat;
aprotische Lösungsmittel wie Dimethylformamid, Dimethylacetamid, Dimethylsulfoxid oder N-Methylpyrrolidinon oder Mischungen der genannten Lösungsmittel einsetzt, oder Phasentransferkatalysatoren wie Quaternäre Ammonium oder
Phosphoniumsalze, beispielsweise Dimethylditetradecylammoniumbromid, Benzyl-triethyl-ammonium-chlorid, 3-Methyl-trioctylammoniumchlorid,
Tetrabutylammoniumhydrogensulfat, oder Tetrabutylphosphoniumchlorid;
Cronenether oder 4,7,13,16,21,24-HEXAOXA-1,10-DIAZABICYCLO-(8.8.8)HEXACOSANE]; oder Polyethylenglykole einsetzt

4. Verfahren nach einem oder mehreren der Ansprüche 1 bis 3, **dadurch gekennzeichnet, dass** man die Verbindung der Formeln I oder III mit einer Säure wie Salzsäure, Schwefelsäure, Salpetersäure oder Phosphorsäure oder Mischungen der Säuren ausfällt.

5. Verfahren nach einem oder mehreren der Ansprüche 1 bis 4, **dadurch gekennzeichnet, dass** man auf 100 Mol der Verbindung der Formeln II oder IV von 150 Mol bis 300 Mol Essigsäureanhydrid und von 100 Mol bis 550 Mol Natriumhydroxid einsetzt.

6. Verfahren nach einem oder mehreren der Ansprüche 1 bis 5, **dadurch gekennzeichnet, dass** man das Lösungsmittel in einer Menge von 3 kg bis 11 kg bezogen auf 1 kg der Verbindung der Formeln II oder IV, bevorzugt von 4 kg bis 6 kg, einsetzt.

7. Verfahren nach einem oder-mehreren der Ansprüche 1 bis 6, **dadurch gekennzeichnet, dass** die Reaktionstemperatur von -5 °C bis 50 °C, bevorzugt von 0 °C bis 30 °C, insbesondere 10 °C, beträgt.

## Claims

1. A process for producing the compound of the formula I

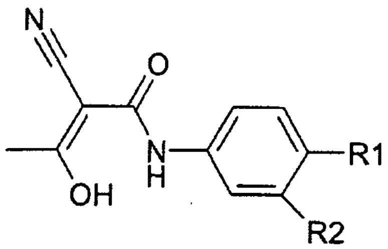

(I)

where

R1 is

a) $-CF_3$,
b) $-O-CF_3$,
c) $-S-CF_3$,
d) -OH,
e) $-NO_2$,
f) halogen,
g) benzyl
h) phenyl,
i) -O-phenyl, k) -CN,
l) -O-phenyl, monosubstituted or poly-substituted with

1) $(C_1-C_4)$-alkyl,
2) halogen,
3) $-O-CF_3$ or
4) $-O-CH_3$, and

R2 is

a) $(C_1-C_4)$-alkyl,
b) halogen or
c) a hydrogen atom,
which comprises reacting a compound of the formula II,

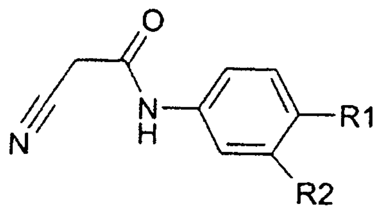

(II)

where R1 and R2 are as defined above,
in the presence of acetic anhydride, at least one solvent and at least one base from the group consisting of alkali metal hydroxides, such as sodium hydroxide or potassium hydroxide, caustic soda in solid form or in the form of lyes, amides, such as sodium amide, alkoxides, such as sodium methoxide or potassium tert-butoxide, organometallic compounds, such as n-butyllithium, or mixtures of the bases, and then isolating the resulting compound of the formula I.

**2.** The process as claimed in claim 1, wherein a compound of the formula III

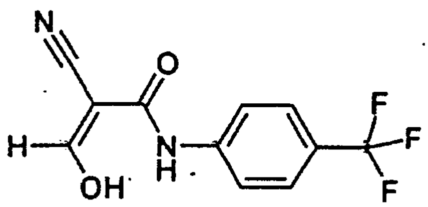

**(III)**

is prepared from a compound of the formula IV

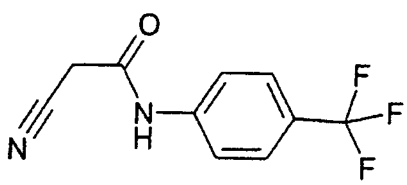

(IV)

and the resulting compound of the formula III is then isolated.

**3.** The process as claimed in claim 1 or 2, wherein the solvent used is water,
ketone solvents, such as acetone, methyl ethyl ketone, diethoxymethane or methyl isobutyl ketone;
halogenated hydrocarbons, such as dichloromethane;
alcohols, such as ethanol, isopropanol or n-butanol;
ethers, such as diisopropyl ether, diethoxymethane, or diethylene glycol dimethyl ether;
hydrocarbons, such as toluene;

esters, such as ethyl acetate;
aprotic solvents, such as dimethylformamide, dimethylacetamide, dimethyl sulfoxide or N-methylpyrrolidinone or mixtures of said solvents, or phase-transfer catalysts, such as quaternary ammonium or phosphonium salts, for example dimethylditetradecylammonium bromide, benzyltriethylammonium chloride, 3-methyltrioctylammonium chloride, tetrabutylammonium hydrogen sulfate, or tetrabutylphosphonium chloride;
crown ethers or 4,7,13,16,21,24-HEXAOXA-1,10-DIAZABICYCLO-(8.8.8)HEXACOSANE]; or polyethylene glycols.

**4.** The process as claimed in one or more of claims 1 to 3, wherein the compound of the formulae I or III is precipitated out using an acid such as hydrochloric acid, sulfuric acid, nitric acid or phosphoric acid, or mixtures of the acids.

**5.** The process as claimed in one or more of claims 1 to 4, wherein from 150 mol to 300 mol of acetic anhydride and from 100 mol to 550 mol of sodium hydroxide are used per 100 mol of the compound of the formulae II or IV.

**6.** The process as claimed in one or more of claims 1 to 5, wherein the solvent is used in an amount of from 3 kg to 11 kg, based on 1 kg of the compound of the formulae II or IV, preferably from 4 kg to 6 kg.

**7.** The process as claimed in one or more of claims 1 to 6, wherein the reaction temperature is from -5°C to 50°C, preferably from 0°C to 30°C, in particular 10°C.

**Revendications**

**1.** Procédé pour la production du composé de formule I,

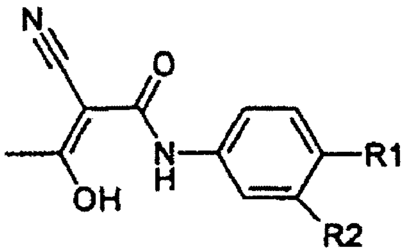

(I)

où

R1 représente

a) $-CF_3$,
b) $-O-CF_3$,
c) $-S-CF_3$,
d) -OH,
e) $-NO_2$,
f) halogène,
g) benzyle,
h) phényle,
i) -O-phényle,
k) -CN,
l) -O-phényle, monosubstitué ou polysubstitué par

1) $(C_1-C_4)$-alkyle,
2) halogène,
3) $O-CF_3$ ou
4) $-O-CH_3$, et

R2 représente

a) $(C_1-C_4)$ -alkyle,

b) halogène ou
c) un atome d'hydrogène,
qui est **caractérisé en ce qu'**on transforme un composé de formule II

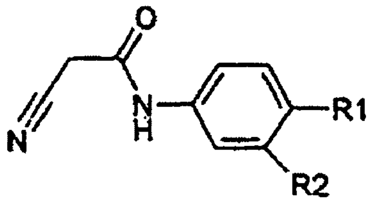
(II)

où R1 et R2 sont définis comme ci-dessus,
en présence d'un anhydride d'acide acétique, d'au moins un solvant et d'au moins une base du groupe formé par les hydroxydes de métal alcalin, tels que l'hydroxyde de sodium ou l'hydroxyde de potassium,
la soude caustique sous forme solide ou sous forme de lessives, les amides tels que l'amide sodique,
les alcoolates tels que le méthylate de sodium ou le tert-butylate de potassium,
les composés organométalliques tels que le n-butyllithium ou
des mélanges des bases, puis on isole le composé de formule I formé.

2. Procédé selon la revendication 1, **caractérisé en ce qu'**on prépare un composé de formule III

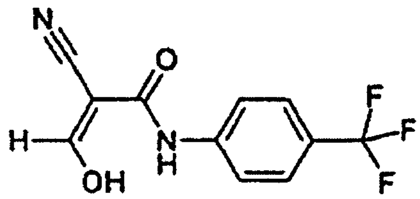
(III)

à partir d'un composé de formule IV

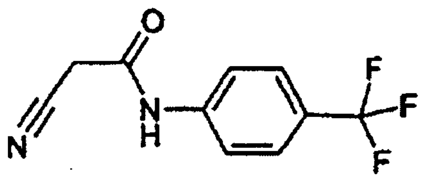
(IV)

puis on isole le composé de formule III formé.

3. Procédé selon la revendication 1 ou 2, **caractérisé en ce qu'**on utilise comme solvant de l'eau, des solvants cétoniques tels que l'acétone, la méthyléthylcétone, le diéthoxyméthane ou la méthylisobutylcétone ;
des hydrocarbures halogénés tels que le dichlorométhane ;
des alcools tels que l'éthanol, l'isopropanol ou le n-butanol ;
des éthers tels que le diisopropyléther, le diéthoxyméthane ou le diéthylèneglycoldiméthyléther ; des hydrocarbures tels que le toluène ;
des esters tels que l'acétate d'éthyle ;
des solvants aprotiques, tels que le diméthylformamide, le diméthylacétamide, le diméthylsulfoxyde ou la N-méthylpyrrolidinone ou des mélanges des solvants mentionnés,
ou des catalyseurs de transfert de phase tels que des sels d'ammonium quaternaire ou des sels de phosphonium, par exemple le bromure de diméthylditétradécylammonium, le chlorure de benzyltriéthylammonium, le chlorure de 3-méthyltrioctylammonium, l'hydrogénosulfate de tétrabutylammonium ; ou le chlorure de tétrabutylphosphonium ; des éther-couronnes ou le 4,7,13,16,21,24-HEXAOXA-1,10-DIAZABICYCLO-(8.8.8)HEXACOSANE] ;
ou des polyéthylèneglycols.

4. Procédé selon l'une ou plusieurs des revendications 1 à 3, **caractérisé en ce qu'**on précipite le composé des formules I ou III avec un acide tel que l'acide chlorhydrique, l'acide sulfurique, l'acide nitrique ou l'acide phosphorique ou des mélanges des acides.

5. Procédé selon l'une ou plusieurs des revendications 1 à 4, **caractérisé en ce qu'**on utilise, pour 100 moles du composé des formules II ou IV, 150 moles à 300 moles d'anhydride de l'acide acétique et 100 moles à 550 moles d'hydroxyde de sodium.

6. Procédé selon l'une ou plusieurs des revendications 1 à 5, **caractérisé en ce qu'**on utilise le solvant en une quantité de 3 kg à 11 kg par rapport à 1 kg du composé des formules II ou IV, de préférence de 4 kg à 6 kg.

7. Procédé selon l'une ou plusieurs des revendications 1 à 6, **caractérisé en ce que** la température de réaction est de -5 à 50°C, de préférence de 0 à 30°C, en particulier de 10°C.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 5679709 A
- US 5519042 A
- US 5700822 A
- DE 1900947